(19) Europäisches Patentamt
European Patent Office
Office européen des brevets

(11) Veröffentlichungsnummer : **0 041 614**
**B1**

(12) # EUROPÄISCHE PATENTSCHRIFT

(45) Veröffentlichungstag der Patentschrift : **28.12.83**

(51) Int. Cl.³ : **C 09 C 1/02**, C 09 C 3/10, A 61 K 6/06, A 61 F 1/00

(21) Anmeldenummer : **81103431.3**

(22) Anmeldetag : **06.05.81**

(54) **Mit organischem Material beschichtetes Bariumsulfat-Pulver.**

(30) Priorität : **17.05.80 DE 3018966**

(43) Veröffentlichungstag der Anmeldung : **16.12.81 Patentblatt 81/50**

(45) Bekanntmachung des Hinweises auf die Patenterteilung : **28.12.83 Patentblatt 83/52**

(84) Benannte Vertragsstaaten : **DE FR GB IT**

(56) Entgegenhaltungen :
**DE-A- 1 592 903**
**GB-A- 1 278 413**
**US-A- 4 124 562**
**CHEMICAL ABSTRACTS, Band 85, Nr. 14, 4. Oktober 1976, Seite 321, Nr. 99135n Columbus, Ohio, U.S.A. G.P. PEARSON et al.: "Effect of barium sulfate on strength of bone cement"**

(73) Patentinhaber : **BAYER AG**
**Zentralbereich Patente, Marken und Lizenzen**
**D-5090 Leverkusen 1, Bayerwerk (DE)**

(72) Erfinder : **Ritter, Helmut, Dr.**
**Bodelschwinghstrasse 16**
**D-4150 Krefeld 1 (DE)**
Erfinder : **Walkowiak, Michael, Dr.**
**Albertus-Magnus-Strasse 10**
**D-5090 Leverkusen 1 (DE)**
Erfinder : **Podszun, Wolfgang, Dr.**
**Wolfskaul 4**
**D-5000 Koeln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 041 614 B1

Jouve, 18, rue St-Denis, 75001 Paris, France

## Mit organischem Material beschichtetes Bariumsulfat-Pulver

Die vorliegende Erfindung betrifft ein mit organischem Material beschichtetes $BaSO_4$-Pulver, das leicht in Monomersysteme, wie sie für die Herstellung von Knochenzementen oder Zahnfüllmassen verwendet werden, eingearbeitet werden kann und nach beendeter Polymerisation ein mechanisch beanspruchbares, röntgenopaques Kunstoffprodukt liefert.

Für den Arzt ist es mitunter notwendig, mittels Röntgenaufnahmen eine genaue Kenntnis über Lage und Sitz einer Zahnfüllung oder eines Knochenzementes zu erlangen.

Es lag daher nahe, das seitlangem bekannte, nahezu wasserunlösliche $BaSO_4$-Pulver den üblichen pastenartigen Monomer/Füllstoff-Mischungen [z. B. denjenigen gemäß DE-A-2 816 823] zuzusetzen und dann durch geeignete Initiierung gemeinsam auszuhärten. Es hat sich jedoch leider gezeigt, daß vermutlich aufgrund der polaren Oberfläche der $BaSO_4$-Kristalle, kein ausreichender Verbund mit der umgebenden Polymermatrix erfolgt, wodurch die Festigkeit und die Abrasionsbeständigkeit der mit $BaSO_4$ gefüllten Kunststoffsysteme noch sehr zu wünschen übrig lassen.

Es ist bereits bekannt, daß durch Silanisierung oder auch durch organische Beschichtung von anorganischen Füllstoffen oder Pigmenten gewisse Verbesserungen bei der Einarbeitung in Kunststoffe oder Harzsysteme erzielt werden können [DE-A-2 347 423].

Es hat sich jedoch gezeigt, daß die dort beschriebenen Verfahren bzw. die verwendeten Beschichtungssubstanzen Füllstoffe liefern, die bei einer Verwendung in Zahnfüllmassen oder Knochenzementen den hohen Anforderungen hinsichtlich minimaler Toxizität, extremer Festigkeit und Härte sowie Abrasionsbeständigkeit nicht vollständig gerecht werden.

Es ist leicht einsehbar, daß nicht optimale mechanische Werte die Gebrauchsdauer bzw. Belastbarkeit von Zahnfüllmassen und Knochenzementen deutlich herabsetzen. Die Verwendung niedermolekularer Beschichtungssubstanzen kann außerdem bei Diffusion zu einer unerwünschten Belastung des Organismus führen. Das in der Praxis häufig angewendete Silanisierungsverfahren führt zwar zu einer verbesserten Benetzbarkeit des Füllstoffs, jedoch geht damit bei den $BaSO_4$-enthaltenden Polymerendprodukten ein Abfall der mechanischen Werte, besonders der Druckfestigkeit, einher.

Das Ziel der Vorliegenden Erfindung besteht daher in der Bereitstellung von organisch modifizierten $BaSO_4$-Füllstoffen mit guter Benetzbarkeit und besonders mit einer positiven Beeinflussung der mechanischen Eigenschaften des gehärteten Polymersystems, wobei aufgrund der medizinischen Anwendung keinerlei niedermolekulare Komponenten, die toxisch sein können, durch Diffusion freigesetzt werden sollen.

Es wurde nun überraschenderweise gefunden, daß durch eine homogene Beschichtung von $BaSO_4$-Partikeln mit geeigneten Polyacrylatsystemen eine raschere Dispergierbarkeit in medizinisch verwendbare Monomersysteme ermöglicht wird und nach der Aushärtung röntgenopake Materialien mit verbesserten mechanischen Werten erhalten werden.

Gegenstand der Erfindung ist ein mit organischen Polymeren modifiziertes $BaSO_4$-Pulver, welches dadurch gekennzeichnet ist, daß die Oberfläche des $BaSO_4$-Materials in homogener Weise bedeckt ist mit einem wasserlöslichen Copolymeren aus

a) 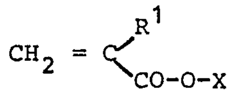

b) 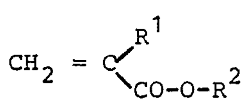 und, gegebenenfalls,

c) 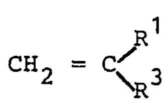

wobei

$R^1$ unabhängig voneinander Wasserstoff oder Methyl,

$R^2$ Alkyl bzw. Cycloalkyl mit 1 bis 10 C-Atomen oder Hydroxyalkyl mit 2 bis 4 C-Atomen,

$R^3$ Alkyl mit 1 bis 5 C-Atomen, Sulfonat, Acetat, Acetamid, Nitril, Carbonamid oder Phenyl und

X Na, K oder Ba bedeuten,

wobei die Säurezahl des Copolymeren 60 bis 350, vorzugsweise 120 bis 260, das Molekulargewicht 1 000 bis 100 000, vorzugsweise 5 000 bis 30 000 und die Menge der Polymerdeckschicht 0,1 bis 5 Gew.%, vorzugsweise 0,5 bis 2 Gew.%, bezogen auf das beschichtete Material, betragen.

Ein weiterer Gegenstand der Erfindung ist ein Verfahren zur Herstellung eines mit organischen Polymeren modifizierten $BaSO_4$-Pulvers, welches dadurch gekennzeichnet ist, daß man eine wäßrige Lösung eines erfindungsgemäßen Copolymeren einer Dispersion von $BaSO_4$-Pulver in Wasser unter Rühren zugibt, absaugt, gegebenenfalls wäscht und schließlich trocknet und mahlt.

In DE-A 1 592 903 wird ein Verfahren zur Behandlung von Pigmenten mit einem makromolekularen Material auf Basis von Acrylsäure und Acrylsäureestern beschrieben, das organophile und hydrophile Gruppen enthält. Das verwendete makromolekulare Material unterscheidet sich von den erfindungsgemäß eingesetzen Copolymeren durch die Natur der hydrophilen Gruppen (gemäß DE-A 1 592 903 wird die freie Säure verwendet oder ein Ammoniumsalz, wobei in der Regel beim Trocknen des beschichteten Pigments die Säureform zurückgebildet wird). Durch die Beschichtung der Pigmente mit dem makromolekularen Material sollen Glanz- und Deckkraft von Lacken, welche die Pigmente enthalten, verbessert werden. Eine Verbesserung des mechanischen Eigenschaftsniveaus wird hingegen nicht angesprochen, sie wird auch in keiner Weise durch die Tatsache der verbesserten optischen Eigenschaften nahegelegt. Es fehlt auch jegliche Anregung für die Bereitstellung eines $BaSO_4$-Pulvers für medizinische Einsatzzwecke.

In GB-A 1 278 413 werden Zahnfüllmassen mit Füllstoffen auf Basis von $SiO_2$, Silicat oder Keramik beschrieben, die silanisiert und anschließend mit Polymethylmethacrylat beschichtet wurden. Der Literaturstelle ist ebenfalls kein Hinweis auf die Beschichtung von $BaSO_4$ zu entnehmen, darüberhinaus wird auch nichts darüber ausgesagt, daß das zur Beschichtung verwendete Polymer die oben erwähnten hydrophilen Gruppen enthalten soll. Das Beschichtungsverfahren gemäß GB-A 1 278 413 läßt sich auch nicht auf $BaSO_4$ übertragen, da die Silanisierungsreaktion (vermutlich infolge Fehlens von Hydroxylgruppen in der Kristalloberfläche) nicht zur Knüpfung chemischer Bindungen führt. Im untenstehenden Beispiel 3 wird gezeigt, daß die Silanisierung von $BaSO_4$ zu keiner Verbesserung der mechanischen Eigenschaften von hieraus hergestellten Kunststoffen führt.

Die Herstellung der erfindungsgemäß als Oberflächenbeschichtung eingesetzten Polymerkomponente erfolgt nach bekannten Verfahren in organischen Lösungsmitteln, die auch Wasser enthalten können, oder bevorzugt nach bekannten Emulsionspolymerisationsverfahren in wäßrigem Medium unter Verwendung von Tensiden, wie Alkylsulfonaten und einem Radikalinitiator, wie $K_2S_2O_8$, bei Temperaturen von 50 bis 90°, bevorzugt bei 75 bis 85°. Zur Molekulargewichtsregelung werden bekannte Verbindungen, wie z. B. Mercaptoethanol, Dodecylmercaptan oder Allylalkohol bis zu 4 Gew.-% zugeführt. Bevorzugt wird Dodecylmercaptan in einer Menge von 0,5-1 Gew.-% verwendet. Das mittlere Molekulargewicht der resultierenden Polymeren bewegt sich je nach Reglermenge im Bereich von ca. 1 000 bis 100 000 ; bevorzugt werden jedoch Polymere mit einem mittleren Molekulargewicht von 5 000 bis 30 000 eingesetzt. Der Polymeranteil der Emulsion beträgt 10 bis 40 Gew.-%, bevorzugt 20 bis 30 Gew.-%.

Geeignete Monomere zur Herstellung der erfindungsgemäßen Beschichtungspolymerisate sind z. B. Acrylnitril, Styrol, α-Methylstyrol, Ethylen, Propylen, Vinylacetat, Vinylpropionat, N-Vinylacetamid, (Meth)-acrylamid, (Meth)acrylsäure, Itaconsäure, (Meth)acrylsäureester mit 1-10 C-Atomen in der Esterkomponente, 2-Hydroxy-ethyl(meth)acrylat, 2-Hydroxy-propyl(meth)-acrylat, Vinylsulfonsäure.

Besonders geeignete Beschichtungspolymerisate im Sinne der Erfindung sind Terpolymere aus Methacrylsäure, Butylacrylat und 2-Hydroxypropylmethacrylat.

Als Bariumsulfatpulver wird ein für Röntgenuntersuchungen geeignetes Handelsprodukt verwendet, das die Bezeichnung « chemisch rein » trägt und durch ein Fällverfahren in einer mittleren Teilchengröße von 0,01-10 μ, bevorzugt 0,1-1,5 μ, gemessen durch Sedimentationsanalyse, erhalten wird.

Die homogene Beschichtung der $BaSO_4$-Partikel erfolgt zunächst durch Dispergieren des $BaSO_4$-Pulvers in Wasser unter schnellem Rühren zu einer Maische, wobei die Primärpartikel in hohem Anteil aus den Agglomerisaten gebildet werden. Der Feststoffgehalt der Maische kann zwischen etwa 10-50 Gew.-% eingestellt werden, bevorzugt zwischen 15 und 30 Gew.-%. Unter Rühren wird eine wäßrige Lösung des Polymeren zugetropft. Der pH-Wert der Polymerlösung liegt im Bereich von 6,0 bis 12,0, bevorzugt 7,0 bis 9,0 ; die Konzentration beträgt 5 bis 20 Gew.-% bevorzugt 8-15 Gew.-%.

Anschließend wird filtriert, mit entsalztem Wasser gewaschen und bei 100 ca. 200 °C getrocknet und gemahlen.

Es werden Bariumsulfat-Pulver erhalten, die leicht für sich allein oder aber gemeinsam mit weiteren Füllstoffen, wie beispielsweise silanisiertes Siliziumdioxid oder feinteiliges Polymethylmethacrylat, mit Monomeren, wie z. B. Methylmethacrylat, Hexandiolbisacrylat, Methacrylsäureaddukte an Bisphenol A-diglycidether (Bis G MA), zu pastenartigen Mischungen vearbeitet werden können, die nach Aushärtung durch bekannte Initiatorsysteme, ein röntgenopaques mechanisch verbessertes Kunststoffsystem liefern.

Beispiel 1

Herstellung der organischen Beschichtungssubstanzen

1,0 g $C_{18}$-Alkylsulfonat wird in 250 ml Wasser zusammen mit 1 g t-Dodecylmercaptan vorgegeben. Unter $O_2$-Ausschluß wird bei 70-80° eine Mischung aus :

a) 20 g Methacrylsäure, 30 g Butylacrylat und 50 g 2-Hydroxypropylmethacrylat ;
b) 40 g Methacrylsäure und 60 g Butylacrylat ;
c) 20 g Methacrylsäure und 80 g Butylacrylat ;
d) 30 g Methacrylsäure, 50 g Butylacrylat und 20 g Acrylnitril ;
e) 20 g Methacrylsäure, 70 g Butylacrylat und 10 g Acrylamid

langsam über einen Zeitraum von 1-2 h zugetropft. Gleichzeitig wird portionsweise eine Lösung aus 0,5 g $K_2S_2O_8$ in 50 ml Wasser während ca. 2 h zugegeben. Nach beendeter Polymerisation werden Latices mit einem Feststoffgehalt von 25 Gew.-% erhalten. Durch Zugabe von 5-10 %iger Natronlauge resultiert eine klare, viskose Lösung.

Beispiel 2

$BaSO_4$-Beschichtung

100 g $BaSO_4$-Pulver wird in 400 ml Wasser angemaischt. Anschließend werden jeweils 4 g der in Beispiel 1 hergestellten Latices a) bis e), in wenig verdünnter Natronlauge gelöst (pH ≈ 7-8) und der $BaSO_4$-Maische unter Rühren langsam zugetropft. Es wird noch ca. 5 Minuten nachgerührt, filtriert, mit 1 Liter Wasser gewaschen und schließlich getrocknet und gemahlen. Im Waschwasser ist kein organischer Kohlenstoff nachweisbar, so daß quantitative Beschichtung von 1 Gew.-% erfolgt ist.

Silanisierung von $BaSO_4$ (Vergleichsversuch)

In einen 1 l Dreihalskolben mit Flügelrührer, Rückflußkühler und Thermometer werden

400 ml Aceton
198 g $BaSO_4$
2,0 g Silan A 174 (UCC) α-Methacryloxypropyltrimethoxysilan
0,5 g Methacrylsäure

gegeben und 2 h unter Rückfluß gekocht (Badtemp. ≈ 70 °C). Anschließend wird der Rückflußkühler durch einen Intensivkühler ersetzt und das Aceton abdestilliert und bei 20° bis 100 °C im Trockenschrank getrocknet. Der Rückstand wird durch ein 150 µ Sieb gegeben.

Beispiel 3

Herstellung von härtbaren Pasten

300 g Bariumsulfat, 15 g silanisiertes amorphes Siliziumdioxid mit einer BET-Oberfläche von 170 m²/g, 60 g Bis G MA, 40 g Triethylenglycoldimethacrylat und 1 g Benzoylperoxid werden in einem Kneter 30 Minuten lang zu einer Paste vermischt, wobei während der letzten 10 Minuten ein Vakuum von 20 Torr angelegt wird.

Mechanische Werte der nach den Beispielen 1-3 hergestellten Materialien

Die Pasten werden in Form von 4 mm starken Platten bei 100 °C 30 Minuten ausgehärtet. Aus diesen Platten wurden Prüfkörper mit den Abmessungen 50 × 6 × 4 mm und 12 × 6 × 4 mm für den Biege- und Druckversuch herausgesägt. Die einzelnen Messungen wurden jeweils an 5 Probekörpern durchgeführt.

Biegeversuch nach DIN 53 452

| Probe | Bariumsulfat | Biegefestigkeit in N/mm² |
|---|---|---|
| 1 | unbehandelt | 48,32 ± 3.61 |
| 2 | silanisiert | 46,54 ± 2.10 |
| 3 | behandelt mit 1a | 58,82 ± 1.06 |
| 4 | behandelt mit 1b | 61,72 ± 1.85 |
| 5 | behandelt mit 1c | 56,58 ± 3.64 |

Druckversuch nach DIN 53 452

| Probe | Stauchspannung bei 1,5 % in $Nmm^2$ | Druckfestigkeit in $N/mm^2$ |
|---|---|---|
| 1 | 195.09 ± 7.04 | 215.57 ± 12.61 |
| 2 | 164.31 ± 8.39 | 192.82 ± 16.58 |
| 3 | 242.64 ± 10.37 | 266.43 ± 13.35 |
| 4 | 220.84 ± 7.52 | 244.90 ± 8.80 |
| 5 | 224.32 ± 4.25 | 252.87 ± 15.66 |

## Ansprüche

1. Mit organischen Polymeren modifiziertes $BaSO_4$-Pulver, dadurch gekennzeichnet, daß die Oberfläche des $BaSO_4$-Materials in homogener Weise bedeckt ist mit einem wasserlöslichen Copolymeren aus

a) 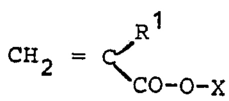

b) 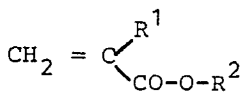 und, gegebenenfalls,

c) 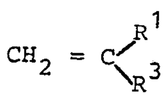

wobei
$R^1$ unabhängig voneinander Wasserstoff oder Methyl,
$R^2$ Alkyl bzw. Cycloalkyl mit 1 bis 10 C-Atomen oder Hydroxyalkyl mit 2 bis 4 C-Atomen,
$R^3$ Alkyl mit 1 bis 5 C-Atomen, Sulfonat, Acetat, Acetamid, Nitril, Carbonamid oder Phenyl und
X Na, K oder Ba bedeuten,
wobei die Säurezahl des Copolymeren 60 bis 350, vorzugsweise 120 bis 260, das Molekulargewicht 1 000 bis 100 000, vorzugsweise 5 000 bis 30 000 und die Menge der Polymerdeckschicht 0,1 bis 5 Gew.-%, vorzugsweise 0,5 bis 2 Gew.-%, bezogen auf das beschichtete Material, betragen.

2. Modifiziertes $BaSO_4$-Pulver nach Anspruch 1, dadurch gekennzeichnet, daß Komponente a des Copolymeren Methacrylsäure ist.

3. Modifiziertes $BaSO_4$-Pulver gemäß Anspruch 1 oder 2, dadurch gekennzeichnet, daß Komponente b des Copolymeren Butylacrylat ist.

4. Modifiziertes $BaSO_4$-Pulver gemäß Anspruch 1 oder 2, dadurch gekennzeichnet, daß Komponente b des Copolymeren eine Mischung von Butylacrylat und 2-Hydroxypropylmethacrylat ist.

5. Modifiziertes $BaSO_4$-Pulver gemäß Anspruch 1 bis 4, dadurch gekennzeichnet, daß Komponente c des Copolymeren ein Monomer aus der Gruppe Acrylnitril, Styrol, α-Methylstyrol, Ethylen, Propylen, Vinylacetat, Vinylpropionat, N-Vinylacetamid, Acrylamid, Methacrylamid und Vinylsulfonsäure ist.

6. Modifiziertes $BaSO_4$-Pulver nach Anspruch 1 bis 5, dadurch gekennzeichnet, daß das Copolymer aus Methacrylsäure, Butylacrylat und 2-Hydroxypropylmethacrylat zusammengesetzt ist.

7. Modifiziertes $BaSO_4$-Pulver nach Anspruch 1 bis 6, dadurch gekennzeichnet, daß das $BaSO_4$ eine mittlere Teilchengröße von 0,01 bis 10 μ, vorzugsweise 0,1 bis 1,5 μ, aufweist.

8. Verfahren zur Herstellung eines mit organischen Polymeren modifizierten $BaSO_4$-Pulvers, dadurch gekennzeichnet, daß man eine wäßrige Lösung eines Copolymeren nach Anspruch 1 einer Dispersion von $BaSO_4$-Pulver in Wasser unter Rühren zugibt, absaugt, gegebenenfalls wäscht und schließlich trocknet und mahlt.

9. Verwendung von modifizierten $BaSO_4$-Füllstoffen gemäß Anspruch 1 bis 7 in Zahnfüllmassen.

10. Verwendung von modifizierten $BaSO_4$-Füllstoffen gemäß Anspruch 1 bis 7 in Knochenzementen.

## Claims

1. A $BaSO_4$ powder modified with organic polymers, characterised in that the surface of the $BaSO_4$

material is homogeneously coated with a water-soluble copolymer of

a) 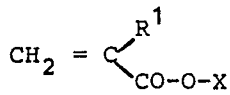

b) 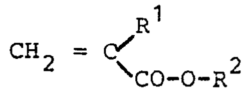 and, optionally,

c) 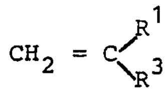

wherein

the symbols $R_1$ independently of one another denote hydrogen or methyl,

$R^2$ denotes alkyl or cycloalkyl with 1 to 10 C atoms or hydroxyalkyl with 2 to 4 C atoms,

$R^3$ denotes alkyl with 1 to 5 C atoms, sulphonate, acetate, acetamide, nitrile, carboxamide or phenyl and

X denotes Na, K or Ba,

the acid number of the copolymer being 60 to 350, preferably 120 to 260, the molecular weight being 1,000 to 100,000, preferably 5,000 to 30,000 and the quantity of the covering polymer layer being 0.1 to 5% by weight, preferably 0.5 to 2% by weight, based on the coated material.

2. Modified $BaSO_4$ powder according to Claim 1, characterised in that component a of the copolymer is methacrylic acid.

3. Modified $BaSO_4$ powder according to Claim 1 or 2, characterised in that component b of the copolymer is butyl acrylate.

4. Modified $BaSO_4$ powder according to Claim 1 or 2, characterised in that component b of the copolymer is a mixture of butyl acrylate and 2-hydroxypropyl methacrylate.

5. Modified $BaSO_4$ powder according to Claim 1 to 4, characterised in that component c of the copolymer is a monomer from the group comprising acrylonitrile, styrene, α-methylstyrene, ethylene, propylene, vinyl acetate, vinyl propionate, N-vinyl-acetamide, acrylamide, methacrylamide and vinyl sulphonic acid.

6. Modified $BaSO_4$ powder according to Claim 1 to 5, characterised in that the copolymer is composed of methacrylic acid, butyl acrylate and 2-hydroxypropyl methacrylate.

7. Modified $BaSO_4$ powder according to Claim 1 to 6, characterised in that the $BaSO_4$ has an average particle size of 0.01 to 10 μ, preferably 0.1 to 1.5 μ.

8. Process for producing a $BaSO_4$ powder modified with organic polymers, characterised in that an aqueous solution of a copolymer according to Claim 1 is added to a dispersion of $BaSO_4$ powder in water, whilst stirring, and the solid is filtered off, if necessary washed, and finally dried and ground.

9. Use of modified $BaSO_4$ fillers according to Claim 1 to 7 in dental filling compositions.

10. Use of modified $BaSO_4$ fillers according to Claim 1 to 7 in bone cements.

**Revendications**

1. Poudre de $BaSO_4$ modifiée avec des polymères organiques, caractérisée en ce que la surface du $BaSO_4$ est recouverte de façon homogène d'un copolymère hydrosoluble de

a) 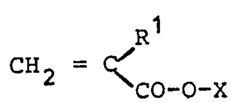

b) 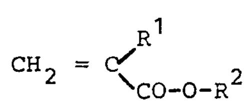 et, le cas échéant,

c) 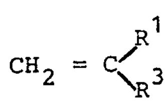

où

$R^1$ représente, indépendamment, l'hydrogène ou le groupe méthyle,

$R^2$ est un groupe alkyle ou cycloalkyle ayant 1 à 10 atomes de carbone ou un groupe hydroxyalkyle ayant 2 à 4 atomes de carbone,

$R^3$ est un groupe alkyle ayant 1 à 5 atomes de carbone, sulfonate, acétate, acétamide, nitrile, carboxamide ou phényle et

X représente Na, K ou Ba,

l'indice d'acide du copolymère ayant une valeur de 60 à 350, de préférence de 120 à 260, le poids moléculaire allant de 1 000 à 100 000, de préférence de 5 000 à 30 000, et la quantité de couche de polymère d'enrobage étant de 0,1 à 5 % en poids, de préférence de 0,5 à 2 % en poids par rapport à la matière enrobée.

2. Poudre de $BaSO_4$ modifiée suivant la revendication 1, caractérisée en ce que le composant a du copolymère est l'acide méthacrylique.

3. Poudre de $BaSO_4$ modifiée suivant la revendication 1 ou 2, caractérisée en ce que le composant b du copolymère est l'acrylate de butyle.

4. Poudre de $BaSO_4$ modifiée suivant la revendication 1 ou 2, caractérisée en ce que le composant b du copolymère est un mélange d'acrylate de butyle et de méthacrylate de 2-hydroxypropyle.

5. Poudre de $BaSO_4$ modifiée suivant les revendications 1 à 4, caractérisée en ce que le composant c du copolymère est un monomère du groupe acrylonitrile, styrène, α-méthylstyrène, éthylène, propylène, acétate de vinyle, propionate de vinyle, N-vinylacétamide, acrylamide, méthacrylamide et acide vinylsulfonique.

6. Poudre de $BaSO_4$ modifiée suivant les revendications 1 à 5, caractérisée en ce que le copolymère est formé d'acide méthacrylique, d'acrylate de butyle et de méthacrylate de 2-hydroxypropyle.

7. Poudre de $BaSO_4$ modifiée suivant les revendications 1 à 6, caractérisée en ce que le $BaSO_4$ a une grosseur moyenne de particules de 0,01 à 10 $\mu$, de préférence de 0,1 à 1,5 $\mu$.

8. Procédé de production d'une poudre de $BaSO_4$ modifiée par un polymère organique, caractérisé en ce qu'on ajoute une solution aqueuse d'un copolymère suivant la revendication 1 à une dispersion de poudre de $BaSO_4$ dans l'eau en agitant, on filtre à la trompe, on lave le cas échéant et, finalement, on sèche et on broie.

9. Utilisation de charges de $BaSO_4$ modifiées suivant les revendications 1 à 7, dans des matières pour l'obturation dentaire.

10. Utilisation de charges de $BaSO_4$ modifiées suivant les revendications 1 à 7, dans des ciments pour les os.